# EUROPEAN PATENT SPECIFICATION

(11) **EP 1 601 752 B1**
(45) Date of publication and mention of the grant of the patent: **13.01.2010**
(21) Application number: 04757493.4
(22) Date of filing: 12.03.2004
(51) Int. Cl.: C11D 3/50, A61K 8/49, C07C 49/00, C07C 49/105

(54) **MACROCYCLIC MUSKS**
MAKROCYCLISCHE MOSCHEN
MUSCS MACROCYCLIQUES

(30) Priority: 13.03.2003 US 453969 P
(43) Date of publication of application: 07.12.2005
(73) Proprietor: Flexitral, Inc., Chantilly, VA 20151 (US)
(72) Inventor: TURIN, Luca, London NW1 7NB (GB)
(74) Representative: Bawden, Peter Charles
(86) International application number: PCT/US2004/007977
(87) International publication number: WO 2004/083357

(56) References cited:
- US-A- 3 778 483
- US-A- 4 885 397
- US-A- 5 948 812
- US-A- 6 034 052
- K. SATO, M. AOKI: "a practical method for alcohol oxidation with aqueous hydrogen peroxide under organic solvent-and halide free conditions." BULLETIN OF THE CHEMICAL SOCIETY OF JAPAN., vol. 72, no. 10, 1999, pages 2287-2306, XP002412777 JPCHEMICAL SOCIETY OF JAPAN, TOKYO.

## Description

### Field of the Invention

The present invention relates generally to the field of fragrances. More particularly, the present invention relates to improved derivatives that provide perfumes and other articles with a musk like odor. These derivatives find utility in applications requiring the themes supplied by the macrocyclic musks. The invention relates to novel macrocyclic musks and mixtures of macrocyclic musks and their use as perfume materials for application to a variety of substrates.

### Description of the Prior Art

Musk fragrances are well known and much used perfume ingredients in perfumes for a large range of products. For example, perfumes for application in laundry detergents, fabric softeners, rinse conditioners and other products intended for use on textile fibers primarily contain musk fragrances of the class of macrocyclic and polycyclic musks. Musk fragrances are present in many perfume oils in not inconsiderable amounts. Accordingly, the annual worldwide requirement for musk fragrances is several thousand tons. By far the largest part is provided by the so-called polycyclic aromatic musk fragrances. It has become known that polycyclic aromatic musk fragrances (well known examples of this class are marketed under various tradenames such as Extralide, Tonalide, Traseolide, Galaxolide, etc.) can only be biodegraded with difficulty and consequently, being extremely lipophilic compounds, exhibit bioaccumulative behavior, i.e. they are able to accumulate in the fatty tissue of organisms. In the perfume industry, there is therefore, a pressing need for biodegradable musk fragrances which are suitable both in terms of the odiferous properties and also in terms of price as replacements for the polycyclic aromatic compounds. In contrast to the polycyclic aromatic compounds, macrocyclic musk fragrances are regarded as biodegradable (U.S. Pat. No. 6,034,052). Typical such musk fragrances are characterized by a macrocyclic ring having 13 to 17 carbon atoms which carries a ketone or an ester as functional group. Moreover, macrocyclic musk fragrances which carry two functional groups are also known, e.g. 1,7-dioxacycloalkan-8-ones (EP A 884,315).

Macrocyclic musks are also long known in perfumery; well known examples of this class of perfume ingredients are the macrocyclic ketones and macrocyclic lactones (macrolides) or dilactones, e.g., cyclohexadecanone, hexadecanolide, cyclopentadecanone, cyclopentadecanolide and various unsaturated and/or methyl substituted analogues thereof. [Williams, Synthesis 1999, No. 10, 1707-1723]

Improved fragrances and flavorings that have odorant intensities similar to the macrocyclic musks are disclosed. In particular, derivatives that maintain or improve the flavor and/or fragrance characteristics of the macrocyclic musks are disclosed. Also disclosed are methods of making the derivatives, and articles of manufacture including the derivatives.

### SUMMARY OF THE INVENTION

The invention is directed toward macrocyclic musks wherein the C=C double bond has been converted to an oxirane or thiirane. The double bond of the well known macrocyclic musks having the formulae: 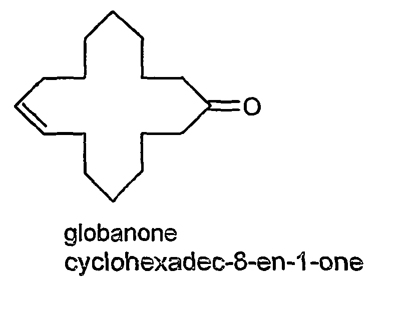 is converted to an oxirane group and then, if desired, to a thiirane group according to the following scheme:

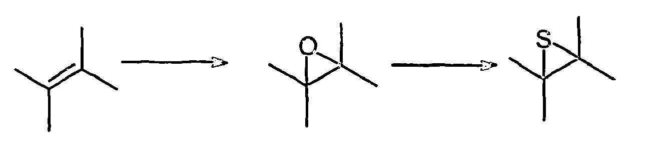

One embodiment of the invention relates to a compound or mixtures of compounds of the formula 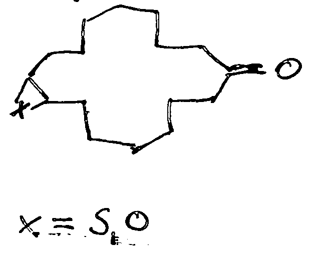 or a mixture comprising it.

A second embodiment of the invention concerns compositions, products, preparations or articles having improved aroma, fragrance or odor characteristics containing as active ingredient a compound or mixture of compounds comprising said compounds of the first embodiment.

Another embodiment of the invention relates to compositions, products, preparations or articles having improved flavor or taste characteristics containing as active ingredient a compound or mixtures of compounds comprising said compounds of the first embodiment.

A further embodiment of the invention concerns methods to confer, improve, enhance or modify the taste or flavor property of a composition, product, preparation or article which comprises adding thereto a flavor effective amount of a compounds or mixtures of compounds comprising said compounds of the first embodiment. containing a methyl substituent on the ring and/or an ether oxygen or thioether sulfur substituting for a CH₂ somewhere in the ring.

A still further embodiment of the invention relates to a method to confer, improve, enhance or modify the aroma, fragrance or odor characteristics of compositions, products, preparations or articles which comprises adding thereto an aroma, fragrance or odor effective amount of a compound or mixture of compounds comprising said compounds of the first embodiment.

Examples of suitable articles of manufacture in which the derivatives of the invention may be incorporated include perfumes and colognes, candles, air fresheners, detergent compositions and disinfectant compositions.

The derivatives of the invention can be used, for example, as fragrances in any applications in which the corresponding macrocyclic musks are applied.

### DETAILED DESCRIPTION OF THE INVENTION

The present invention is predicated on the discovery of improved fragrances and flavorings that have increased odorant and/or flavorant intensity relative to conventional macrocyclic musks. In particular, the compounds of the invention maintain the flavor and/or fragrance characteristics of the analogous macrocyclic musks, while increasing the odor intensity relative thereto. Also disclosed are methods of making the compounds, utilizing the derivatives as flavorants and/or fragrances and compositions, products, preparations and articles incorporating the derivatives. The derivatives of the invention find utility in any application requiring musk themes. The compositions, products, preparations and articles in which the compounds and derivatives of the invention may be incorporated include candles, air fresheners, perfumes, fragrances, colognes, soaps, bath or shower gels, shampoos or other hair care products, cosmetic preparations, body odorants, deodorants or antiperspirants, liquid or solid fabric detergents or softeners, bleach products (hypochlorites), disinfectants, all-purpose household or industrial cleaners, foods, flavorings, beverages such as beer and soda, denture cleansers (tablets), flavored orally-delivered products such as lozenges, candies, chewing gums, matrices, pharmaceuticals and the like.

The derivatives of the invention can be viewed as macrocyclic musks thereof wherein the double bond therein has been replaced by an oxirane or thiirane group. The novel derivatives of the invention are macrocyclic ketones having the following formula:

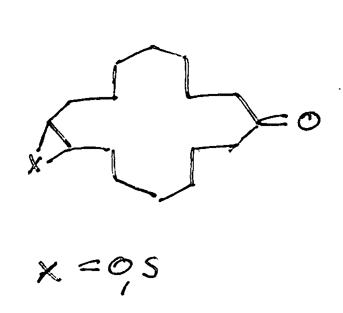

The derivatives of the invention can be prepared according to the methods described in Bouda, N., et al (1989) Synthesis ofEpoxides from Thiiranes in the presence of Thiourea. Synthetic Communications 199:491-500.

### Reference EXAMPLE 1

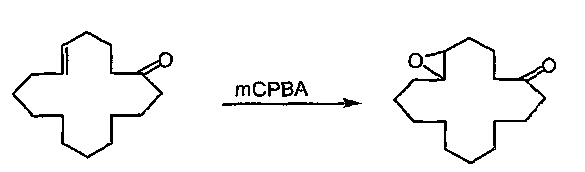

In a 500-mL round bottom flask containing velvione (5-Cyclohexadecen-1-one, 5 g) in dichloromethane (200 mL) was added mCPBA (8.0 g, 50-85%) portionwise over 45 minutes and the solution stirred for 5 hours. The mixture was washed with a solution of saturated Na₂SO₃ (70 mL), followed by washing with brine (3x70 mL). The organic phase was dried over IVa₂SO₄, filtered and the solvent evaporated *in vacuo* to give the pure desired product as a colorless oil (6.0 g )

### Reference EXAMPLE 2

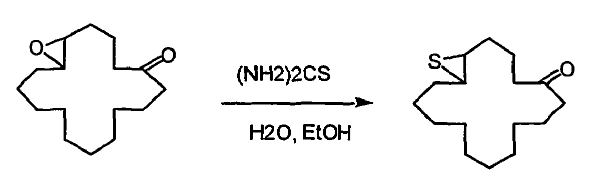

To the epoxide product from example 1 in ethanol (10 mL) and water (6 mL) was added thiourea (1.6 g) and the solution stirred overnight under a N₂ atmosphere. The solvent was evaporated *in vacuo*. Flash column chromatography from the mixture using dichloromethane/ethyl acetate (3:2) afforded the desired thiirane as colorless oil.

The derivatives of the invention can be included in virtually any article of manufacture that can include conventional macrocyclic musks, or for that matter, other fragrances, whether natural or artificial. Examples include bleach, detergents, flavorings and fragrances, beverages, including alcoholic beverages, and the like. The macrocyclic musk derivatives can be used in applications like soaps, shampoos, body deodorants and antiperspirants, solid or liquid detergents for treating textiles, fabric softeners, detergent compositions and/or all-purpose cleaners for cleaning dishes or various surfaces, for both household and industrial use. Of course, the compounds can be used in other current uses in perfumery, namely the perfuming of soaps and shower gels, hygiene or hair-care products, as well as of body deodorants, air fresheners and cosmetic preparations, and even in fme perfumery, namely in perfumes and colognes. The products of the invention also find utility in foods, flavorings, beverages such as beer and soda, denture cleansers (tablets), flavored orally-delivered products such as lozenges, candies, chewing gums, matrices, pharmaceuticals and the like. These uses are described in more detail below.

The compounds can be used as perfuming ingredients, as single compounds or as mixture thereof, preferably at a range of at least about 30% by weight of the perfume composition, more preferably at a range of at least about 60% by weight of the composition. The compounds can even be used in their pure state or as mixtures, without added components. The olfactive characteristics of the individual compounds are also present in mixtures thereof, and mixtures of these compounds can be used as perfuming ingredients: This may be particularly advantageous where separation and/or purification steps can be avoided by using compound mixtures.

In all cited applications, the macrocyclic musk derivatives can be used alone, in admixture with each other, or in admixture with other perfuming ingredients, solvents or adjuvants of current use in the art. The nature and the variety of these co-ingredients do not require a more detailed description here, which, moreover, would not be exhaustive, and the person skilled in the art will be able to choose the latter through its general knowledge and as a function of the nature of the product to be perfumed and of the desired olfactive effect.

These perfuming ingredients typically belong to chemical classes as varied as alcohols, aldehydes, ketones, esters, ethers, acetates, nitrites, terpene hydrocarbons, sulfur- and nitrogen containing heterocyclic compounds, as well as essential oils of natural or synthetic origin. A large number of these ingredients described in reference textbooks such as the book of S. Arctander, Perfume and Flavor Chemicals, 1969, Montclair, N.J., USA, or its more recent versions, or in other works of similar nature.

The proportions in which the macrocyclic musk derivatives can be incorporated in the various products vary within a large range of values. These values depend on the nature of the article or product that one desires to perfume and the odor effect searched for, as well as on the nature of the co-ingredients in a given composition when the compounds are used in admixture with perfuming co-ingredients, solvents or adjuvants of current use in the art.

As an example, the macrocyclic musk derivatives are typically present at concentrations between about 0.1 and about 10%, or even more, by weight of these compounds relative to the weight of the perfuming composition in which they are incorporated. Far lower concentrations than those mentioned above can be used when the compounds are directly applied for perfuming the various consumer products cited beforehand.

The compounds may be used in detergents containing bleaching agents and activators such as, for example, tetraacetylethylenediamine (TAED), hypohalites, in particular hypochlorite, peroxygenated bleaching agents such as, for example, perborates, etc. The compounds can also be used in body deodorants and antiperspirants, for example, those containing aluminum salts. These embodiments are described in more detail below.

In addition to the derivatives described herein, the compositions herein include a detersive surfactant and optionally, one or more additional detergent ingredients, including materials for assisting or enhancing cleaning performance, treatment of the substrate to be cleaned, or to modify the aesthetics of the detergent composition (e.g., perfumes, colorants, dyes, etc.). Non-limiting examples of synthetic detersive surfactants useful herein typically at levels from about 0.5% to about 90%, by weight, include the conventional C1-18 alkyl benzene sulfonates ("LAS") and primary, branch-chain and random CIO-20 alkyl sulfates ("AS"), and the like.

Preferred compositions incorporating only synthetic detergents have a detergent level of from about 0.5% to 50%. Compositions containing soap preferably comprise from about 10% to about 90% soap.

The compositions herein can contain other ingredients such as enzymes, bleaches, fabric softening agents, dye transfer inhibitors, suds suppressors, and chelating agents, all well known within the art.

The macrocyclic musk derivatives described herein can be incorporated into beverages and impart various flavorings to the beverages. The beverage composition can be a cola beverage composition, and can also be coffee, tea, dairy beverage, fruit juice drink, orange drink, lemon-lime drink, beer, malt beverages, or other flavored beverage. The beverages can be in liquid or powdered form. The beverage compositions can also include one or more flavoring agents; artificial colorants; vitamin additives; preservatives; caffeine additives; water; acidulants; thickeners; buffering agents; emulsifiers; and or fruit juice concentrates.

Artificial colorants which may be used include caramel color, yellow 6 and yellow 5. Useful vitamin additives include vitamin B2, vitamin B6, vitamin B12, vitamin C (ascorbic acid), niacin, pantothenic acid, biotin and folic acid. Suitable preservatives include sodium or potassium benzoate. Salts which may be used include sodium, potassium and magnesium chloride. Exemplary emulsifiers are gum arabic and purity gum, and a useful thickener is pectin. Suitable acidulants include citric, phosphoric and malic acid, and potential buffering agents include sodium and potassium citrate.

In one embodiment, the beverage is a carbonated cola beverage. The pH is generally about 2.8 and the following ingredients can be used to make the syrup for these compositions: Flavor Concentrate, including one or more of the derivatives described herein (22.22 ml), 80% Phosphoric Acid (5.55 g), Citric Acid (0.267 g), Caffeine (1.24 g), artificial sweetener, sugar or com syrup (to taste, depending on the actual sweetener) and Potassium Citrate (4.07 g). The beverage composition can be prepared, for example, by mixing the foregoing syrup with carbonated water in a proportion of 50 ml syrup to 250 ml of carbonated water.

Flavored food and pharmaceutical compositions including one or more of the derivatives described herein can also be prepared. The derivatives can be incorporated into conventional foodstuffs using techniques well known to those of skill in the art. Alternatively, the derivatives can be incorporated within polymeric particles, which can, in turn, be dispersed within and/or over a surface of an orally-deliverable matrix material, which is usually a solid or semi-solid substrate. When used in chewable compositions, the derivatives can be released into the orally-deliverable polymeric matrix material as the composition is chewed and held in the mouth, thus prolonging the flavor of the composition. In the case of dried powders and mixes, the flavor can be made available as the product is consumed or be released into the matrix material as the composition is further processed. When two flavors are combined with the polymeric particles, the relative amounts of the additives can be selected to provide simultaneous release and exhaustion of the compounds.

In one embodiment, the flavored composition includes an orally-deliverable matrix material; a plurality of water insoluble polymeric particles dispersed in the orally-deliverable matrix material, where the polymeric particles individually define networks of internal pores and are non-degradable in the digestive tract; and one or more derivatives as described herein entrapped within the internal pore networks. The derivatives are released as the matrix is chewed, dissolved in the mouth, or undergoes further processing selected from the group consisting of liquid addition, dry blending, stirring, mixing, heating, baking, and cooking. The orally-deliverable matrix material can be selected from the group consisting of gums, latex materials, crystallized sugars, amorphous sugars, fondants, nougats, jams, jellies, pastes, powders, dry blends, dehydrated food mixes, baked goods, batters, doughs, tablets, and lozenges.

A flavorless gum base can be combined with a macrocyclic musk derivative or other suitable derivative as described herein to a desired flavor concentration. Typically, a blade mixer is heated to about 11 OF, the gum base is preheated so that it is softened, and the gum base is then added to the mixer and allowed to mix for approximately 30 seconds. The flavored derivative is then added to the mixer and mixed for a suitable amount of time. The gum can be then removed from the mixer and rolled to stick thickness on waxed paper while warm.

In one embodiment, the derivatives described herein are incorporated into a system which can release a fragrance in a controlled manner. These include substrates such as air fresheners, laundry detergents, fabric softeners, deodorants, lotions, and other household items. The fragrances are generally one or more derivatives of essential oils as described herein, each present in different quantities. U.S. Pat. No. 4,587,129, the contents of which are hereby incorporated by reference in their entirety, describes a method for preparing gel articles which contain up to 90% by weight of fragrance or perfume oils. The gels are prepared from a polymer having a hydroxy (lower alkoxy) 2-alkeneoate, a hydroxy (lower alkoxy) lower alkyl 2-alkeneoate, or a hydroxy poly (lower alkoxy)lower alkyl 2-alkeneoate and a polyethylenically unsaturated crosslinking agent. These materials have continuous slow release properties, i.e., they release the fragrance component continuously over a long period of time. Advantageously, all or a portion of those derivatives that include an aldehyde group can be modified to include an acetal group, which can cause the formulations to release fragrance over a period of time as the acetal hydrolyzes to form the aldehyde compound.

## Claims

1. A compound obtainable by converting the C=C double bond of 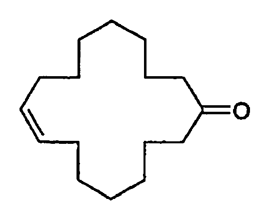 to oxirane or thiirane.

2. A composition, product, preparation or article comprising a compound of claim 1 as a fragrance ingredient.

3. The composition, product, preparation or article of claim 2, wherein the compound is present in an amount of at least 30 percent by weight.

4. The composition, product, preparation or article of claim 2, wherein the compound is present in an amount of at least 60 percent by weight.

5. A composition, product, preparation or article of claim 3 in the form of a perfume, fragrance or cologne, a soap, a bath or shower gel, a shampoo or other hair care product, a cosmetic preparation, a body odorant, deodorant or antiperspirant, an air freshener, a liquid or solid fabric detergent or softener, bleach product, disinfectant or an all-purpose household or industrial cleaner.

6. A perfuming composition, product, preparation or article of claim 5, wherein the compound or mixture of compounds is present in admixture with other perfuming ingredients, solvents, or adjuvants of current use in the art.

7. A composition, product, preparation or article of claim 6 in the form of a body odorant, deodorant or antiperspirant wherein the compound or mixture of compounds is in admixture with other body odorant, deodorant or antiperspirant ingredients, solvents or adjuvants.

8. A perfumed article according to claim 5, in the form of a perfume or cologne, a soap, a bath or shower gel, a shampoo or other hair care product, a cosmetic preparation, a body deodorant or antiperspirant, an air freshener, a fabric detergent or softener or an all-purpose household cleaner.

9. A composition, product, preparation or article of claim 8 in the form of a perfume wherein the compound or mixture of compounds is in admixture with other perfuming ingredients, solvents or adjuvants.

10. A body deodorant or antiperspirant composition, product, preparation or article, of claim 5.

11. A body deodorant or antiperspirant composition, product, preparation or article, of claim 10, wherein the compound or mixture of compounds is present in admixture with other perfuming ingredients, solvents, or adjuvants of current use in the art.

12. A detergent composition, product, preparation or article of claim 5.

13. A detergent composition, product, preparation or article of claim 12 wherein the compound or mixture of compounds is in admixture with detergent ingredients, solvents or adjuvants.

14. A bleach composition, product, preparation or article of claim 5.

15. A bleach composition, product, preparation or article of claim 13 wherein the compound or mixture of compounds is in admixture with bleach ingredients, solvents or adjuvants.

16. A composition, product, preparation or article of claim 5 in the form of a disinfectant.

17. The disinfectant composition, product, preparation or article of claim 16 wherein the compound or mixture of compounds is in admixture with disinfectant ingredients, solvents or adjuvants.

18. A composition, product, preparation or article comprising a compound or mixture of compounds as defined in claim 1 as a flavour or taste ingredient.

19. A composition, product, preparation or article of claim 18 in the form of a beverage.

20. A beverage composition, product, preparation or article of claim 19 wherein the compound or mixture of compounds is in admixture with beverage ingredients, solvents or adjuvants.

21. A composition, product, preparation or article of claim 18 in the form of a flavoring.

22. A flavoring composition, product, preparation or article of claim 21 wherein the compound or mixture of compounds is in admixture with other flavoring ingredients, solvents or adjuvants.

23. A composition, product, preparation or article of claim 18 in the form of a food wherein the compound or mixture of compounds is in admixture with food ingredients, solvents or adjuvants.

24. A food composition, product, preparation or article of claim 23 wherein the compound or mixture of compounds is in admixture with food ingredients, solvents or adjuvants.

25. A composition, product, preparation or article of claim 18 in the form of a chewing gum.

26. A chewing gum composition, product, preparation or article of claim 25 wherein the compound or mixture of compounds is in admixture with chewing gum ingredients, solvents or adjuvants.

27. A composition, product, preparation or article of claim 18 in the form of a pharmaceutical.

28. A pharmaceutical composition, product, preparation or article of claim 27 wherein the compound or mixture of compounds is in admixture with a pharmaceutical ingredient, and solvents or adjuvants.

29. A composition, product, preparation or article of claim 18 in the form of an orally-deliverable matrix material.

30. A composition, product, preparation or article of claim 29 wherein the compound or mixture of compounds is in admixture with a matrix material ingredient, and solvents or adjuvants.

31. A method to confer, improve, enhance or modify the taste or flavor property of a composition, product, preparation or article which comprises adding thereto a flavor effective amount of a compound or mixture of compounds as defined in claim 1.

32. The method of claim 31 wherein said composition, product, preparation or article is in the form of a beverage.

33. The method of claim 31 wherein said composition, product, preparation or article is in the form of a flavoring.

34. The method of claim 31 wherein said composition, product, preparation or article is in the form of a food.

35. The method of claim 31 wherein said composition, product, preparation or article is in the form of a chewing gum.

36. The method of claim 31 wherein said composition, product, preparation or article is in the form of a pharmaceutical.

37. The method of claim 31 wherein said composition, product, preparation or article is in the form of an orally deliverable matrix.

38. A method to confer, improve, enhance or modify the aroma, fragrance or odor characteristics of a composition, product, preparation or article which comprises adding thereto an aroma, fragrance or odor effective amount of a compound or mixture of compounds as defined in claim 1.

39. The method of claim 38 wherein said composition, product, preparation or article is in the form of a perfume.

40. The method of claim 38 wherein said composition, product, preparation or article is in the form of a body odorant, deodorant or antiperspirant.

41. The method of claim 38 wherein said composition, product, preparation or article is in the form of a detergent.

42. The method of claim 38 wherein said composition, product, preparation or article is in the form of a beach product.

43. The method of claim 38 wherein said composition, product, preparation or article is in the form of a disinfectant.

44. An article of manufacture comprising packaging material and an aroma, odor, fragrance, taste or flavor enhancing agent contained within said packaging material, wherein said agent is effective for the enhancement of the aroma, odor, fragrance, taste or flavor of a composition, preparation, product or article to which it is added, and wherein said packaging material comprises a label which indicates that said agent can be used for enhancing aroma, odor, fragrance, taste or flavor, and wherein said agent is a compound or mixture of compounds as defined in claim 1.

## Patentansprüche

1. Eine Verbindung erhältlich durch Umwandlung der C=C-Doppelbindung von 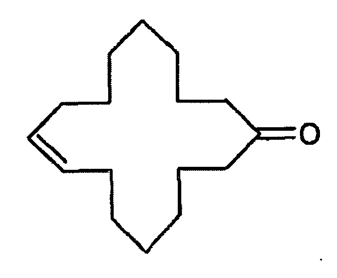 in Oxiran oder Thiiran.

2. Eine Zuammensetzung, Produkt, Zubereitung oder Artikel umfassend eine Verbindung nach Anspruch 1 als ein Duftinhaltsstoff.

3. Zusammensetzung, Produkt, Zubereitung oder Artikel nach Anspruch 2, **dadurch gekennzeichnet, dass** sie die Verbindung mit einem Gehalt von mindestens 30 Gew.-% aufweist.

4. Zusammensetzung, Produkt, Zubereitung oder Artikel nach Anspruch 2, **dadurch gekennzeichnet, dass** sie die Verbindung mit einem Gehalt von mindestens 60 Gew.-% aufweist.

5. Zusammensetzung, Produkt, Zubereitung oder Artikel nach Anspruch 3 in Form eines Parfums, Fragrance oder Cologne, einer Seife, eines Bademittels, eines Duschgels, eines Shampoos oder eines anderen Haarpflegeproduktes, einer kosmetische Zubereitung, eines Körper-Odoriermittels, Deodorants oder Antispirants, eines Lufterfrischers, eines flüssigen oder festen Textilwaschmittels oder eines Weichspülers, eines Bleichproduktes, Desinfektionsmittels oder ein Allzweckhaushalts- oder industriereinigers.

6. Parfumierte Zusammensetzung, Produkt, Zubereitung oder Artikel nach Anspruch 5, **dadurch gekennzeichnet, dass** die Verbindung oder Mischung der Verbindungen als Zusatz mit anderen duftenden Ingredienzen, Lösemitteln, oder üblichen Hilfsmitteln vorliegt.

7. Zusammensetzung, Produkt, Zubereitung oder Artikel nach Anspruch 6 in Form eines Körper-Odoriermittels, Deodorants oder Antispirants, **dadurch gekennzeichnet, dass** die Verbindung oder Mischung der Verbindungen als Zusatz zusammen mit anderen körperodorierend wirkenden, deodorierend wirkenden oder schweißhemmenden Inhaltsstoffen, Lösemitteln oder Hilfsmitteln vorliegt.

8. Ein parfümierter Artikel nach Anspruch 5, in Form eines Parfums oder Colognes, einer Seife, eines Bademittels oder Duschegels, eines Shampoos oder eines anderen Haarpflegeprodukts, einer kosmetischen Zubereitung, eines Körperdeodorants oder Antispirants, eines Lufterfrischers, eines Textilwaschmittels oder Weichspülers oder eines Allzweckhaushaltreinigers.

9. Zusammensetzung, Produkt, Zubereitung oder Artikel nach Anspruch 8 in Form eines Parfums, **dadurch gekennzeichnet, dass** die Verbindung oder Mischung der Verbindungen als Zusatz mit anderen duftenden Ingredentien, Lösemitteln oder Hilfsmitteln vorliegt.

10. Ein Körperdeodorant oder eine Antispirantzusammensetzung, Produkt, Zubereitung oder Artikel nach Anspruch 5.

11. Ein Körperdeodorant oder eine Antispirantzusammensetzung, Produkt, Zubereitung oder Artikel nach Anspruch 10, **dadurch gekennzeichnet, dass** die Verbindung oder Mischung der Verbindungen als Zusatz zusammen mit anderen üblichen Inhaltsstoffen, Lösemitteln oder Hilfsmitteln vorliegt.

12. Detergenszusammensetzung, Produkt, Zubereitung oder Artikel nach Anspruch 5.

13. Eine Detergenszusammensetzung, Produkt, Zubereitung oder Artikel nach Anspruch 12, **dadurch gekennzeichnet, dass** die Verbindung oder Mischung der Verbindungen als Zusatz zusammen mit anderen reinigenden Ingredentien, Lösemitteln oder Hilfsmitteln als Mischung vorliegt.

14. Eine Bleichzusammensetzung, Produkt, Zubereitung oder Artikel nach Anspruch 5.

15. Bleichzusammensetzung, Produkt, Zubereitung oder Artikel nach Anspruch 13, **dadurch gekennzeichnet, dass** die Verbindung oder die Mischung der Verbindungen als Zusatz zusammen mit Bleichmitteln, Lösemitteln oder Hilfsmitteln vorliegt.

16. Eine Zusammensetzung, Produkt, Zubereitung oder Artikel nach Anspruch 5 in Form eines Desinfektionsmittels.

17. Desinfektionszusammensetzung, Produkt, Zubereitung oder Artikel nach Anspruch 16, **dadurch gekennzeichnet, dass** die Verbindung oder Mischung der Verbindungen als Zusatz zusammen mit Desinfektionsmitteln, Lösemitteln oder Hilfsmitteln vorliegt.

18. Zusammensetzung, Produkt, Zubereitung oder Artikel umfassend einer Verbindung oder Mischung der Verbindungen gemäß Anspruch 1 als Duft-, Aroma- oder Geschmackszusatz.

19. Zusammensetzung, Produkt, Zubereitung oder Artikel nach Anspruch 18 in Form eines Getränkes.

20. Eine Getränkezusammensetzung, Produkt, Zubereitung oder Artikel nach Anspruch 19, **dadurch gekennzeichnet, dass** die Verbindung oder Mischungen der Verbindungen als Zusatz zusammen mit Getränkezusatzstoffen, Lösemitteln oder Hilfsmitteln vorliegt.

21. Zusammensetzung, Produkt, Zubereitung oder Artikel nach Anspruch 18 in Form eines Aromastoffs.

22. Eine Aromazusammensetzung, Produkt, Zubereitung oder Artikel nach Anspruch 21, **dadurch gekennzeichnet, dass** die Verbindung oder Mischung der Verbindungen als Zusatz zusammen mit anderen Aromastoffen, Lösemitteln oder Hilfsstoffen vorliegt.

23. Zusammensetzung, Produkt, Zubereitung oder Artikel nach Anspruch 18 in Form eines Nahrungsmittels, **dadurch gekennzeichnet, dass** die Verbindung oder Mischung der Verbindungen als Zusatz zusammen mit Nahrungsmittelbestandteilen, Lösemitteln oder Hilfsmitteln vorliegt.

24. Eine Nahrungsmittelzusammensetzung, Produkt, Zubereitung oder Artikel nach Anspruch 23, **dadurch gekennzeichnet, dass** die Verbindung oder Mischung der Verbindungen als Zusatz zusammen mit Nahrungsmittelzusatzstoffen, Lösemitteln oder Hilfsmitteln vorliegt.

25. Zusammensetzung, Produkt, Zubereitung oder Artikel nach Anspruch 18 in Form eines Kaugummis.

26. Eine Kaugummizusammensetzung, Produkt, Zubereitung oder Artikel nach Anspruch 25, **dadurch gekennzeichnet, dass** die Verbindung oder Mischung der Verbindungen als Zusatz zusammen mit Kaugummünhaltsstoffen, Lösemitteln oder Hilfsmitteln vorliegt.

27. Zusammensetzung, Produkt, Zubereitung oder Artikel nach Anspruch 18 in Form eines Arzneimittels.

28. Eine pharmazeutische Zusammensetzung, Produkt, Zubereitung oder Artikel nach Anspruch 27, **dadurch gekennzeichnet, dass** die Verbindung oder Mischungen der Verbindungen als Zusatz zusammen mit pharmazeutischen Inhaltsstoffen und Lösemitteln oder Hilfsstoffen vorliegt.

29. Zusammensetzung, Produkt, Zubereitung oder Artikel nach Anspruch 18 in Form eines oral zuführbaren Matrixmaterials.

30. Zusammensetzung, Produkt, Zubereitung oder Artikel nach Anspruch 29, **dadurch gekennzeichnet, dass** die Verbindung oder Mischung von Verbindungen zusammen mit einem Matrixmaterialbestandteil und Lösemittels oder Hilfsmittels in einer Mischung vorliegt.

31. Ein Verfahren um einer Zusammensetzung, einem Produkt, einer Zubereitung oder einem Artikels einen Geschmack oder Aromaeigenschaften zu verleihen, zu verbessern, zu verfeinern oder zu modifizieren, umfassend die Zugabe eines zur Aromatisierung effektiven Anteils einer Verbindung oder einer Mischung der Verbindung gemäß Anspruch 1.

32. Verfahren nach Anspruch 31, **dadurch gekennzeichnet, dass** die Zusammensetzung, das Produkt, die Zubereitung oder der Artikel in Form eines Getränkes vorliegt.

33. Verfahren nach Anspruch 31, **dadurch gekennzeichnet, dass** die Zusammensetzung, das Produkt, die Zubereitung oder der Artikel in Form eines Aromastoffs vorliegt.

34. Verfahren nach Anspruch 31, **dadurch gekennzeichnet, dass** die Zusammensetzung, Produkt, Zubereitung oder Artikel in Form eines Nahrungsmittels vorliegt.

35. Verfahren nach Anspruch 31, **dadurch gekennzeichnet, dass** die Zusammensetzung, das Produkt, die Zubereitung oder der Artikel in Form eines Kaugummis vorliegt.

36. Verfahren nach Anspruch 31, **dadurch gekennzeichnet, dass** die Zusammensetzung, das Produkt, die Zubereitung oder der Artikel in Form eines Arzneimittels vorliegt.

37. Verfahren nach Anspruch 31, **dadurch gekennzeichnet, dass** die Zusammensetzung, das Produkt, die Zubereitung oder der Artikel in Form einer oral verabreichbaren Matrix vorliegt.

38. Ein Verfahren um einer Zusammensetzung einem Produkt, einer Zubereitung oder einem Artikel eine Aroma-, Duft- oder Geruchscharakteristik zu verleihen, zu verbessern, zu verfeinern oder sie zu modifizieren, umfassend hierzu die Zugabe eines aromatisierenden, duftenden oder geruchseffektiven Anteils einer Verbindung oder Mischung von Verbindungen gemäß Anspruch 1.

39. Verfahren nach Anspruch 38, **dadurch gekennzeichnet, dass** die Zusammensetzung, das Produkt, die Zubereitung oder der Artikel in Form eines Parfums vorliegt.

40. Verfahren nach Anspruch 38, **dadurch gekennzeichnet, dass** die Zusammensetzung, das Produkt, die Zubereitung oder der Artikel in Form eines Körper-Odoriermittels, Deodorants oder Antispirants vorliegt.

41. Verfahren nach Anspruch 38, **dadurch gekennzeichnet, dass** die Zusammensetzung, das Produkt, die Zubereitung oder der Artikel in Form eines Reinigungsmittels vorliegt.

42. Verfahren nach Anspruch 38, **dadurch gekennzeichnet, dass** die Zusammensetzung, das Produkt, die Zubereitung oder der Artikel in Form eines Bleichproduktes vorliegt.

43. Verfahren nach Anspruch 38, **dadurch gekennzeichnet, dass** die Zusammensetzung, das Produkt, die Zubereitung oder der Artikel in Form eines Desinfektionsmittels vorliegt.

44. Hergestellter Artikel, umfassend Packmaterial und ein Aroma, Geruch, Duft, Geschmack oder Bukett verbesserndes Mittel enthaltend im Packmaterial, **dadurch gekennzeichnet, dass** das Mittel geeignet ist das Aroma, den Geruch, den Duft, den Geschmack oder das Bukett einer Zusammensetzung, Zubereitung, eines Produktes oder Artikels zu dem es zugegeben wird, zu verbessern, und wobei das Packmaterial ein Etikett bzw. Aufschrift umfasst, das anzeigt, dass das Mittel verwendet werden kann, um das Aroma, den Geruch, das Bukett, den Geschmack oder den Duft zu verbessern, und wobei das Mittel eine Verbindung oder Mischung der Verbindungen nach Anspruch 1 ist.

## Revendications

1. Composé susceptible d'être obtenu en convertissant la double liaison C=C de 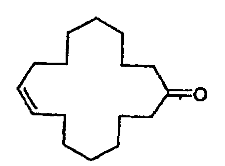 en oxirane ou thiirane.

2. Composition, produit, préparation ou article comprenant un composé selon la revendication 1 en tant qu'ingrédient de fragrance.

3. Composition, produit, préparation ou article selon la revendication 2, dans lesquels le composé est présent en une quantité d'au moins 30 pour cent en poids.

4. Composition, produit, préparation ou article selon la revendication 2, dans lesquels le composé est présent en une quantité d'au moins 60 pour cent en poids.

5. Composition, produit, préparation ou article selon la revendication 3, sous la forme d'un parfum, d'une fragrance ou d'une eau de Cologne, d'un savon, d'un gel pour le bain ou la douche, d'un shampoing ou d'un autre produit pour le soin des cheveux, d'une préparation cosmétique, d'un odorant, déodorant ou antisudorifique corporel, d'un assainisseur d'air, d'un détergent ou assouplissant liquide ou solide pour le linge, d'un produit de blanchiment, d'un désinfectant ou d'un nettoyant ménager ou industriel polyvalent.

6. Composition, produit, préparation ou article parfumant(e) selon la revendication 5, dans lesquels le composé ou le mélange de composés est présent en mélange avec d'autres ingrédients parfumants, des solvants ou des adjuvants d'usage courant dans la technique.

7. Composition, produit, préparation ou article selon la revendication 6 sous la forme d'un odorant, déodorant ou antisudorifique corporel, dans lesquels le composé ou le mélange de composés est en mélange avec d'autres ingrédients odorants, déodorants ou antisudorifiques corporels, des solvants ou des adjuvants.

8. Article parfumé selon la revendication 5, sous la forme d'un parfum ou d'une eau de Cologne, d'un savon, d'un gel pour le bain ou la douche, d'un shampoing ou d'un autre produit pour le soin des cheveux, d'une préparation cosmétique, d'un odorant ou antisudorifique corporel, d'un assainisseur d'air, d'un détergent ou assouplissant pour le linge ou d'un nettoyant ménager polyvalent.

9. Composition, produit, préparation ou article selon la revendication 8, sous la forme d'un parfum dans lequel le composé ou le mélange de composés est en mélange avec d'autres ingrédients parfumants, des solvants ou des adjuvants.

10. Composition, produit, préparation ou article déodorant ou antisudorifique corporel selon la revendication 5.

11. Composition, produit, préparation ou article déodorant ou antisudorifique corporel selon la revendication 10, dans lesquels le composé ou le mélange de composés est présent en mélange avec d'autres ingrédients parfumants, des solvants ou des adjuvants d'usage courant dans la technique.

12. Composition, produit, préparation ou article détergent(e) selon la revendication 5.

13. Composition, produit, préparation ou article détergent(e) selon la revendication 12, dans lesquels le composé ou le mélange de composés est en mélange avec des ingrédients détergents, des solvants ou des adjuvants.

14. Composition, produit, préparation ou article de blanchiment selon la revendication 5.

15. Composition, produit, préparation ou article de blanchiment selon la revendication 13, dans lesquels le composé ou le mélange de composés est en mélange avec des ingrédients de blanchiment, des solvants ou des adjuvants.

16. Composition, produit, préparation ou article selon la revendication 5, sous la forme d'un désinfectant.

17. Composition, produit, préparation ou article désinfectant(e) selon la revendication 16, dans lesquels le composé ou le mélange de composés est en mélange avec des ingrédients désinfectants, des solvants ou des adjuvants.

18. Composition, produit, préparation ou article comprenant un composé ou un mélange de composés tel que défini dans la revendication 1 en tant qu'ingrédient de saveur ou de goût.

19. Composition, produit, préparation ou article selon la revendication 18, sous la forme d'une boisson.

20. Composition, produit, préparation ou article de boisson selon la revendication 19, dans lesquels le composé ou le mélange de composés est en mélange avec des ingrédients de boisson, des solvants ou des adjuvants.

21. Composition, produit, préparation ou article selon la revendication 18, sous la forme d'un agent de saveur.

22. Composition, produit, préparation ou article de saveur selon la revendication 21, dans lesquels le composé ou le mélange de composés est en mélange avec d'autres ingrédients de saveur, des solvants ou des adjuvants.

23. Composition, produit, préparation ou article selon la revendication 18 sous la forme d'un aliment, dans lesquels le composé ou le mélange de composés est en mélange avec des ingrédients alimentaires, des solvants ou des adjuvants.

24. Composition, produit, préparation ou article alimentaire selon la revendication 23, dans lesquels le composé ou le mélange de composés est en mélange avec des ingrédients alimentaires, des solvants ou des adjuvants.

25. Composition, produit, préparation ou article selon la revendication 18, sous la forme d'une gomme à mâcher.

26. Composition, produit, préparation ou article de gomme à mâcher selon la revendication 25, dans lesquels le composé ou le mélange de composés est en mélange avec des ingrédients de gomme à mâcher, des solvants ou des adjuvants.

27. Composition, produit, préparation ou article selon la revendication 18, sous la forme d'un produit pharmaceutique.

28. Composition, produit, préparation ou article pharmaceutique selon la revendication 27, dans lesquels le composé ou le mélange de composés est en mélange avec un ingrédient pharmaceutique, et des solvants ou des adjuvants.

29. Composition, produit, préparation ou article selon la revendication 18, sous la forme d'une matière matricielle délivrable oralement.

30. Composition, produit, préparation ou article selon la revendication 29, dans lesquels le composé ou le mélange de composés est en mélange avec un ingrédient de matière matricielle, et des solvants ou des adjuvants.

31. Procédé pour conférer, améliorer, exalter ou modifier la propriété du goût ou de la saveur d'une composition, d'un produit, d'une préparation ou d'un article, qui comprend l'addition à ceux-ci d'une quantité efficace de saveur d'un composé ou mélange de composés tel que défini dans la revendication 1.

32. Procédé selon la revendication 31, dans lequel lesdits composition, produit, préparation ou article sont sous la forme d'une boisson.

33. Procédé selon la revendication 31, dans lequel lesdits composition, produit, préparation ou article sont sous la forme d'un agent de saveur.

34. Procédé selon la revendication 31, dans lequel lesdits composition, produit, préparation ou article sont sous la forme d'un aliment.

35. Procédé selon la revendication 31, dans lequel lesdits composition, produit, préparation ou article sont sous la forme d'une gomme à mâcher.

36. Procédé selon la revendication 31, dans lequel lesdits composition, produit, préparation ou article sont sous la forme d'un produit pharmaceutique.

37. Procédé selon la revendication 31, dans lequel lesdits composition, produit, préparation ou article sont sous la forme d'une matrice délivrable oralement.

38. Procédé pour conférer, améliorer, exalter ou modifier les caractéristiques d'arôme, de fragrance ou d'odeur d'une composition, d'un produit, d'une préparation ou d'un article, qui comprend l'addition à ceux-ci d'une quantité efficace d'arôme, de fragrance ou d'odeur d'un composé ou mélange de composés tel que défini dans la revendication 1.

39. Procédé selon la revendication 38, dans lequel lesdits composition, produit, préparation ou article sont sous la forme d'un parfum.

40. Procédé selon la revendication 38, dans lequel lesdits composition, produit, préparation ou article sont sous la forme d'un odorant, déodorant ou antisudorifique corporel.

41. Procédé selon la revendication 38, dans lequel lesdits composition, produit, préparation ou article sont sous la forme d'un détergent.

42. Procédé selon la revendication 38, dans lequel lesdits composition, produit, préparation ou article sont sous la forme d'un produit de blanchiment.

43. Procédé selon la revendication 38, dans lequel lesdits composition, produit, préparation ou article sont sous la forme d'un désinfectant.

44. Article manufacturé comprenant un matériau d'emballage et un agent exaltant l'arôme, l'odeur, la fragrance, le goût ou la saveur contenu à l'intérieur dudit matériau d'emballage, dans lequel ledit agent est efficace pour exalter l'arôme, l'odeur, la fragrance, le goût ou la saveur d'une composition, d'une préparation, d'un produit ou d'un article auxquels il est ajouté, et dans lequel ledit matériau d'emballage comprend une étiquette qui indique que ledit agent peut être utilisé pour exalter l'arôme, l'odeur, la fragrance, le goût ou la saveur, et dans lequel ledit agent est un composé ou un mélange de composés tel que défini dans la revendication 1.
